# EUROPEAN PATENT APPLICATION

(11) **EP 3 731 438 A1**
(43) Date of publication of application: **28.10.2020**
(21) Application number: 18891979.9
(22) Date of filing: 21.11.2018
(51) Int. Cl.: H04B 13/00

(54) **DATA COMMUNICATION DEVICE**

(30) Priority: 21.12.2017 JP 2017244665; 21.12.2017 JP 2017244666
(71) Applicant: Panasonic Intellectual Property Management Co., Ltd., Osaka-shi, Osaka 540-6207 (JP)
(72) Inventor: KUMAHARA Minoru, Osaka-shi, Osaka 540-6207 (JP); MINAMIYAMA Masanori, Osaka-shi, Osaka 540-6207 (JP); TOMITA Mitsuru, Osaka-shi, Osaka 540-6207 (JP)
(74) Representative: Vigand, Philippe
(86) International application number: PCT/JP2018/042934
(87) International publication number: WO 2019/123955

(57) **Abstract**

A data communication device to provide can have stable communication quality. Data communication device (11) according to the present disclosure includes a fitting surface and an outer surface opposite from the fitting surface. Data communication device (11) also includes belt (22), buckle (23), a communication electrode, and a communication unit. Belt (22) is joined to buckle (23) The communication electrode is formed at belt (22) or buckle (23) to be exposed at the fitting surface. The communication unit is electrically connected to the communication electrode. Buckle (23) includes a buckle mechanism. Because of this structure, belt (22) can be set to have a right length. Therefore, a reduced clearance can be achieved between the communication electrode and a human body when data communication device (11) is put on. FIG. 2
15
HOT ELECTRODE
16
COLD ELECTRODE
13a
TRANSMITTER
13b
RECEIVER
12
POWER SOURCE
13d
MEMORY
13c
CONTROLLER
19
DISPLAY UNIT
18
OUTPUT I/F

## Description

### TECHNICAL FIELD

The present disclosure relates to a wearable data communication device that is fitted to a part of a human body when used.

### BACKGROUND ART

A conventional wearable data communication device is hereinafter described. The conventional wearable data communication device includes a body case, a communication unit, a communication electrode, a ground electrode, a belt, and a buckle. The communication unit is housed in the body case. The communication electrode comes into contact with a human body and transmits and receives communication signals. The belt is joined to the body case. The ground electrode is formed at a leading portion of the belt. A connection between the ground electrode and the communication unit and a connection between the communication electrode and the communication unit are made by, for example, wires routed inside the belt.

When such a data communication device is put on the human body, the belt is worn around a human body part such as an arm. The belt is fastened with the buckle and thus is kept attached to the human body. Provided near a leading edge of the belt are a plurality of pin holes. The buckle, on the other hand, includes a metal ring and a pin. The pin spans the ring. When the data communication device is put on the human body, with the leading edge of the belt passed through the ring, the pin is inserted into the pin hole that adjusts the belt to a specified length. The data communication device communicates with a communication control device via the human body when the communication control device is touched by, for example, a finger or a palm. To this end, the communication electrode and the ground electrode both touch the human body. In other words, the communication electrode and the ground electrode are both disposed on the same side as the case's surface of contact with the human body.

PTLs 1 and 2 are known examples of prior art literature on a wearable data communication device such as described above as being relevant to the present disclosure.

### Citation List

### Patent Literatures

PTL 1: Unexamined Japanese Patent Publication No. 2006-239716
PTL 2: Unexamined Japanese Patent Publication No. 2003-134009

### SUMMARY OF THE INVENTION

In the case of the above-described conventional data communication device, however, the belt length adjustment is restricted by pin hole position. Therefore, the belt may not be adjusted to a length that allows the communication electrode to touch skin of a wearer. For a typical belt, a belt portion to be joined to a buckle can be cut off so that belt has an adjusted length. However, because of the wires routed inside the belt, a belt portion cannot be cut off for an adjusted length. Consequently, a great clearance may problematically be caused between the communication electrode and the human body, depending on the wearer.

Since the communication electrode and the ground electrode of the above-described conventional data communication device are both disposed on the side that the case touches the human body, there is a close distance between the communication electrode and the ground electrode. Accordingly, signals output from a transmission electrode or signals to be received by the transmission electrode flow toward the ground electrode, thus problematically causing poor communication sensitivity between the data communication device and the communication control device.

The present disclosure overcomes these problems, and an object of the present disclosure is to provide a data communication device that enables a reduced clearance between a communication electrode and a human body and can have stable communication quality together with satisfactory communication sensitivity between the data communication device and a communication control device.

To achieve this object, a data communication device according to one aspect of the present disclosure includes a fitting surface and an outer surface. The outer surface is positioned opposite the fitting surface. The data communication device also includes a buckle, a belt, a communication electrode, and a communication unit. The belt is joined to the buckle. The communication electrode is formed at the belt or the buckle to be exposed at the fitting surface. The communication unit is electrically connected to the communication electrode. In the above structure, the buckle includes a buckle mechanism. The above structure can achieve the intended object.

A data communication device according to another aspect of the present disclosure includes a body, a belt, a power source, a communication unit, a communication electrode, and a ground electrode of plate shape. The body case includes a bottom face and a side face. The belt is joined to the body case at the side face of the body case. The power source is housed in the body case. The communication unit is driven by the power source. The communication electrode is connected to the communication unit and is exposed at the bottom face of the body case. The ground electrode is connected to a ground of the communication unit inside the body. The belt at least partly includes an insulator. The ground electrode includes a second surface that extends out of the body case to face a first plane extending, as an extension of a surface of the communication electrode, beyond the body case. The second surface is covered by the insulator. This structure can achieve the intended object.

As described above, the buckle of the present disclosure has the buckle mechanism, so that length of the belt can be adjusted freely. Because the belt can be set to have a length that is right for a wearer, a reduced clearance can be achieved between the communication electrode and a human body when the wearer puts on the data communication device. As a result, the data communication device provided can have stable communication quality.

The insulator can prevent the ground electrode from directly touching the human body when the data communication device according to the present disclosure is fitted to the human body. Therefore, signals output from a transmission electrode or signals to be received by the transmission electrode do not easily flow toward the ground electrode. Consequently, improved communication sensitivity is achieved between the data communication device and a communication control device.

### BRIEF DESCRIPTION OF DRAWINGS

FIG. 1 is a conceptual diagram of a data communication system according to an exemplary embodiment of the present disclosure.
FIG. 2 is a block diagram of a data communication device according to the exemplary embodiment of the present disclosure.
FIG. 3 is a conceptual diagram of the data communication device according to the exemplary embodiment of the present disclosure.
FIG. 4 is an exploded perspective view of the data communication device according to the exemplary embodiment of the present disclosure.
FIG. 5 is a front view looking at a front face of the data communication device according to the exemplary embodiment of the present disclosure.
FIG. 6 is a right side view of the data communication device according to the exemplary embodiment of the present disclosure.
FIG. 7 is a left side view of the data communication device according to the exemplary embodiment of the present disclosure.
FIG. 8 is a top view of the data communication device according to the exemplary embodiment of the present disclosure.
FIG. 9 is a bottom view of the data communication device according to the exemplary embodiment of the present disclosure.
FIG. 10 is a rear view looking at a fitting surface of the data communication device according to the exemplary embodiment of the present disclosure.
FIG. 11 is a perspective view of the data communication device according to the exemplary embodiment of the present disclosure.
FIG. 12 is a perspective view illustrating the data communication device with a belt fastened according to the exemplary embodiment of the present disclosure.

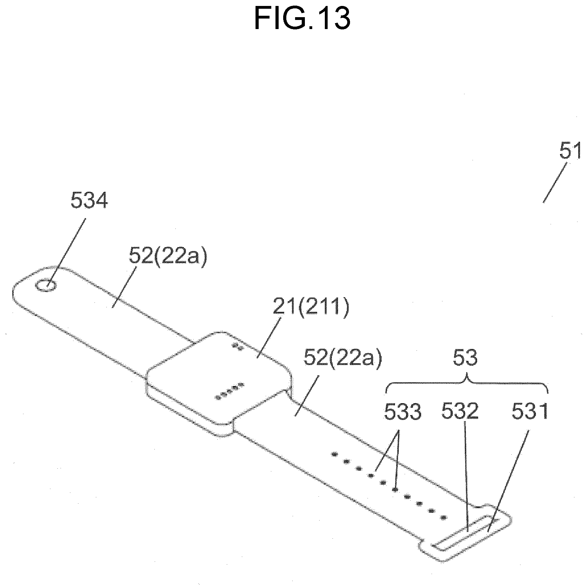

FIG. 13 is a perspective view of a data communication device variation according to the exemplary embodiment of the present disclosure.

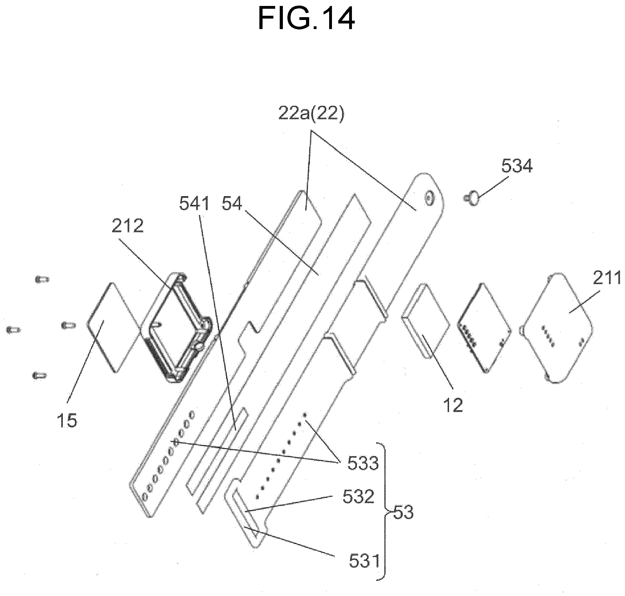

FIG. 14 is an exploded perspective view of the data communication device variation according to the exemplary embodiment of the present disclosure.

### DESCRIPTION OF EMBODIMENT

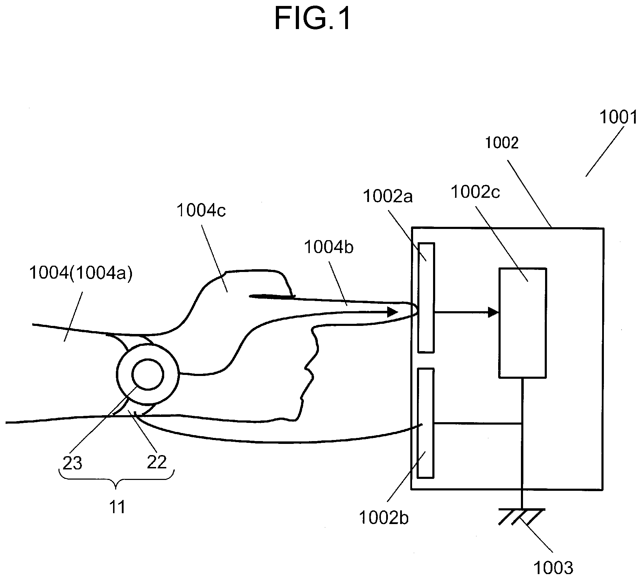

Before a data communication device according to an exemplary embodiment of the present disclosure is described, a data communication system that allows communication using the data communication device is described below. FIG. 1 is a conceptual diagram of data communication system 1001. Data communication system 1001 includes data communication device 11 and communication control device 1002.

Communication control device 1002 includes communication electrode 1002a, ground electrode 1002b, and communication unit 1002c. Communication electrode 1002a is connected to communication unit 1002c. Ground electrode 1002b and a ground terminal (not illustrated) of communication unit 1002c are both connected to ground 1003.

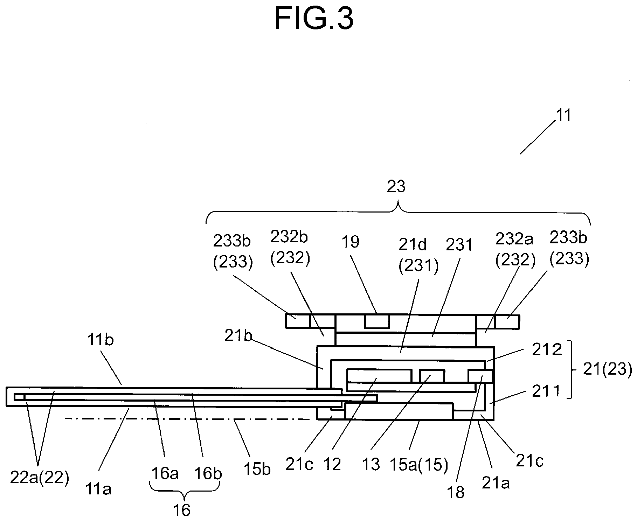

Data communication device 11, on the other hand, is put on wearer 1004. Data communication device 11 is put on wearer's arm 1004a, for example. FIG. 3 is a conceptual diagram of data communication device 11. As illustrated in FIG. 3, data communication device 11 includes fitting surface 11a and outer surface 11b. Outer surface 11b is positioned opposite fitting surface 11a. Data communication device 11 is put on arm 1004a with fitting surface 11a facing arm 1004a. In this case, wearer 1004 touches communication electrode 1002a with hand 1004c. Thus communication is established between data communication device 11 and communication control device 1002. The communication between data communication device 11 and communication control device 1002 is established via a body of wearer 1004. It is to be noted that wearer 1004 only has to touch communication electrode 1002a with a palm, a back of the hand, or finger 1004b. To touch communication electrode 1002a, wearer 1004 may use, for example, arm 1004a, specifically an elbow, so that the part of hand 1004c is not limiting. In all cases, wearer 1004 touches communication electrode 1002a with the body part close to where data communication device 11 is put on. It is also to be noted that something may be interposed between finger 1004b and communication electrode 1002a as long as communication is possible, so that direct touching of communication electrode 1002a by wearer 1004 is not limiting.

When wearer 1004 touches communication electrode 1002a with finger 1004b, data communication device 11 and communication control device 1002 communicate with each other via arm 1004a, hand 1004c, and finger 1004b. In other words, a body part extending from arm 1004a to finger 1004b serves as a path of communication between data communication device 11 and communication control device 1002.

Since data communication device 11 is put on wearer 1004 as described above, data communication device 11 cannot be connected directly to a ground or the like by a ground wire, for example. Therefore, data communication device 11 is generally ungrounded when operating. In other words, data communication device 11 is put in a floating state in terms of potential. Data communication device 11 may be put on a body part such as a leg or a trunk, so that arm 1004a is not limiting.

Wearer 1004 generally touches communication control device 1002 with finger 1004b or hand 1004c when intending to give instructions to communication control device 1002. Therefore, data communication device 11 is put on arm 1004a to be in close proximity to finger 1004b or hand 1004c. Because of this configuration, even when communication control device 1002 is touched by finger 1004b, data communication device 11 is relatively close to ground electrode 1002b. Thus data communication device 11 can be grounded via space between data communication device 11 and ground electrode 1002b.

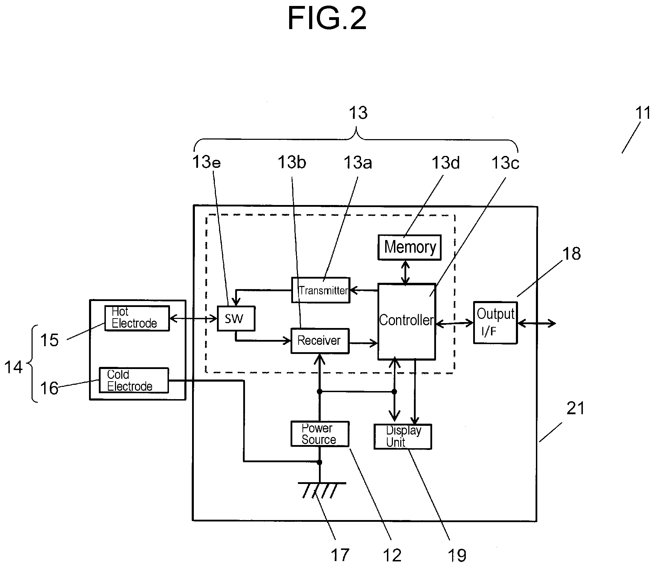

Data communication device 11 according to the present exemplary embodiment is hereinafter described with reference to the drawings. FIG. 2 is a circuit block diagram of data communication device 11. Data communication device 11 includes power source 12, communication unit 13, and communication antenna 14. Communication antenna 14 includes communication electrode (Hot electrode) 15 and planar ground electrode (Cold electrode) 16. Communication unit 13 is driven by power source 12. Communication electrode 15 is connected to communication unit 13. Communication unit 13 transmits communication signals to communication electrode 15. Alternatively, communication unit 13 receives communication signals from communication electrode 15. Ground electrode 16, on the other hand, is connected to ground 17 of communication unit 13.

Data communication device 11 thus configured is put on the body of wearer 1004. Data communication device 11 includes fitting surface 11a and outer surface 11b. Outer surface 11b is positioned opposite fitting surface 11a. Here data communication device 11 is put on with fitting surface 11a facing the body of wearer 1004. When such data communication device 11 is put on, communication electrode 15 faces the human body. In other words, data communication device 11 includes this surface provided with communication electrode 15, and fitting surface 11a refers to this surface. The communication signals from communication electrode 15 thus fitted to wearer 1004 are transmitted to communication control device 1002 via the body of wearer 1004. On the other hand, the communication signals transmitted from communication control device 1002 are received by communication electrode 15 via the body of wearer 1004.

It is preferable that communication electrode 15 directly touch the body of wearer 1004 when data communication device 11 is put on. In this way, communication signal loss can be reduced. Therefore, communication is stable between data communication device 11 and communication control device 1002. It is to be noted that there may be a small clearance between surface 15a of communication electrode 15 and the human body, so that direct touching of the human body by surface 15a is not limiting. A thin dress or the like may also be interposed between the human body and communication electrode 15. In these cases, however, increased stray capacitance between communication electrode 15 and the body of wearer 1004 causes communication signal attenuation. Therefore, a thickness of the dress or a distance across the clearance may be such that a predetermined level of electric field strength or more can be secured at communication electrode 1002a.

FIG. 3 is a conceptual diagram of data communication device 11. Data communication device 11 includes body case 21 and belt 22. Data communication device 11 is held to arm 1004a when belt 22 is fastened. Therefore, belt 22 is worn around arm 1004a and is fastened at its leading end with an immobilizing assembly (described later), thus being fitted and held to arm 1004a. To avoid complication, FIG. 3 does not illustrate ground 17.

Power source 12 and communication unit 13 are housed in body case 21. Body case 21 includes bottom face 21a and side face 21b. Belt 22 is joined to body case 21 at side face 21b of body case 21. The belt at least partly includes insulator 22a. Data communication device 11 is put on with bottom face 21a facing arm 1004a. This means that bottom face 21a of body case 21 is included in the fitting surface.

Communication electrode 15 is provided to be exposed at bottom face 21a of body case 21. On the other hand, one end of ground electrode 16 is connected to ground 17 inside body case 21. Ground electrode 16 extends from inside body case 21 to have its opposite end outside body case 21. Ground electrode 16 is disposed to face plane 15b that extends, as an extension of surface 15a of communication electrode 15, beyond body case 21 (or from bottom face 21a). Ground electrode 16 includes an extending part positioned outside body case 21, and this extending part includes surface 16a facing plane 15b. Surface 16a is covered by insulator 22a. Because of this structure, a surface of insulator 22a that is closer to surface 16a of ground electrode 16 is included in the fitting surface. In other words, a belt side facing plane 15b serves as fitting surface 11a. With body case 21 being put on arm 1004a by means of belt 22, insulator 22a of belt 22 is interposed between (surface 16a of) ground electrode 16 and arm 1004a.

Because of the above structure, insulator 22a can prevent ground electrode 16 from directly touching wearer 1004 when wearer 1004 puts on data communication device 11. Therefore, signals output from a transmission electrode or signals to be received by the transmission electrode do not easily flow toward the ground electrode. Consequently, improved communication sensitivity is achieved between data communication device 11 and communication control device 1002.

Data communication device 11 is hereinafter described in more detail. Power source 12 is preferably a secondary battery. This structure can eliminate battery replacements and thus is advantageous in terms of waterproofness. It is to be noted that power source 12 is not limited to the secondary battery and may be a primary battery.

Communication unit 13 may include those including transmitter 13a, receiver 13b, control circuit 13c, memory 13d, and switch (SW) 13e. Communication electrode 15 is connected to an input and an output terminal of switch 13e. The input terminal of switch 13e is connected to an output of transmitter 13a. The output terminal of switch 13e, on the other hand, is connected to an input of receiver 13b. Switch 13e switches between reception and transmission. For reception, switch 13e electrically connects communication electrode 15 and receiver 13b. For transmission, switch 13e electrically connects communication electrode 15 and transmitter 13a. Memory 13d stores those including identification data (ID data) that identifies, for example, the wearer or the data communication device. Control circuit 13c obtains these data from memory 13d and transmits these data to transmitter 13a. On the other hand, receiver 13b receives, for example, response signals from communication control device 1002. The response signal is indicative of, for example, normal reception of a communication transmitted from data communication device 11 by communication control device 1002.

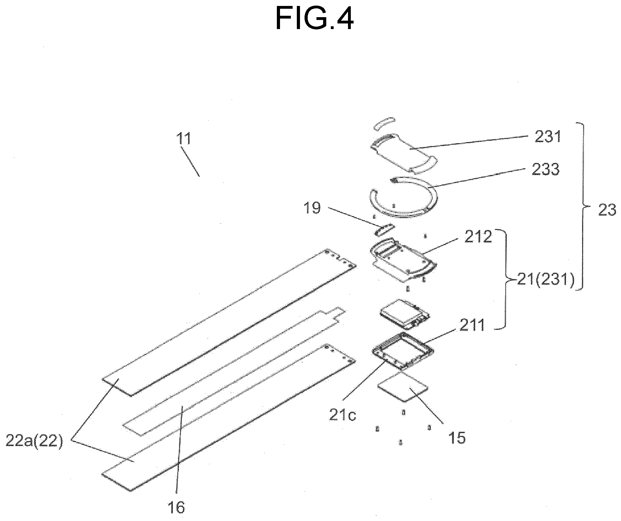

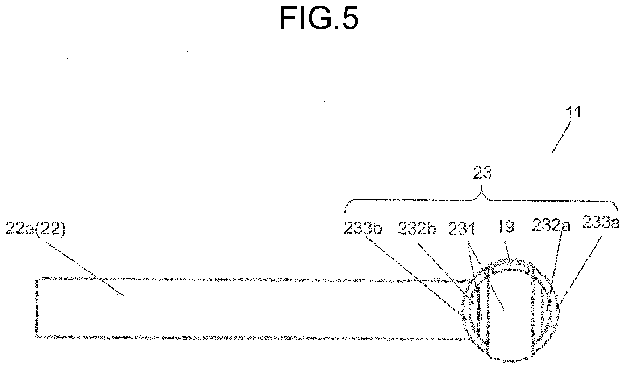

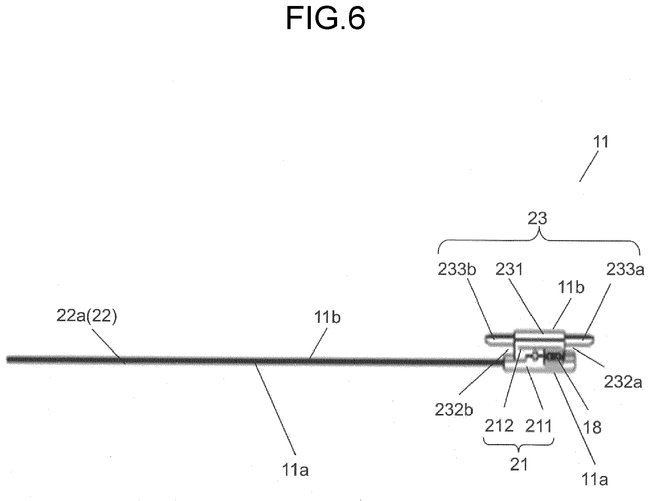

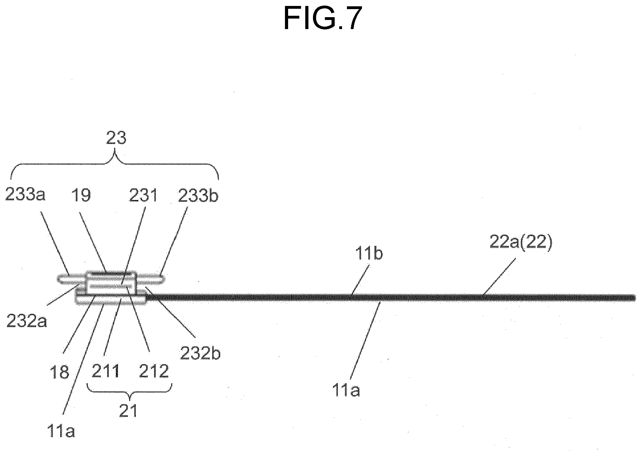

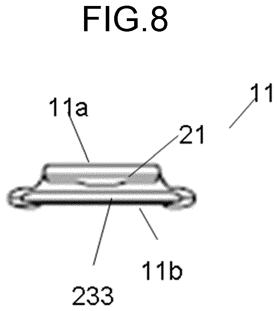

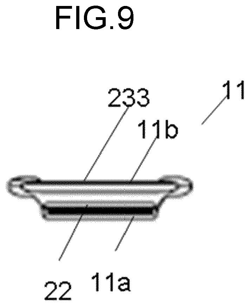

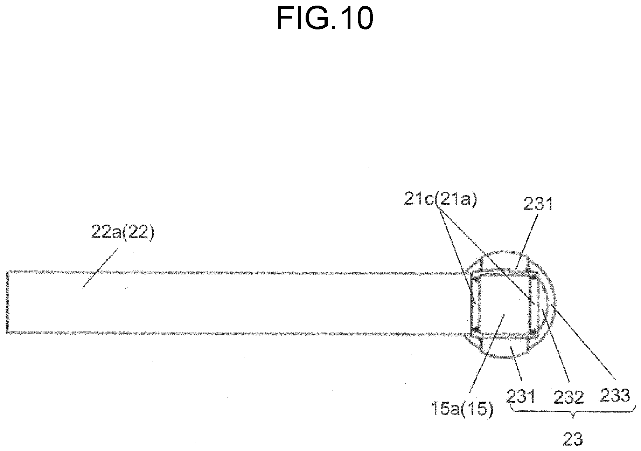

FIG. 4 is an exploded perspective view of data communication device 11. FIG. 5 is a front view looking at outer surface 11b of data communication device 11. FIG. 6 is a right side view of data communication device 11. FIG. 7 is a left side view of data communication device 11. FIG. 8 is a top view of data communication device 11. FIG. 9 is a bottom view of data communication device 11. FIG. 10 is a rear view looking at fitting surface 11a of data communication device 11.

Data communication device 11 may also include external input and output interface (input and output I/F) 18 and display unit 19. External input and output 18 and display unit 19 are housed in body case 21. Display unit 19 is caused by a control signal from control circuit 13c to light up. Display unit 19 lights up when, for example, a communication signal is transmitted normally from transmitter 13a. Alternatively, display unit 19 lights up when a response signal is received from receiver 13b. This configuration enables the wearer to be aware of completion of normal communication between data communication device 11 and communication control device 1002. Data communication device 11 can also import external data for transmission to communication control device 1002. If data communication device 11 includes external input and output interface 18, power source 12 can also be supplied with power from an external device via a Universal Serial Bus (USB) cable to be charged.

Since belt 22 is worn around arm 1004a, belt 22 is made of a flexible material. Also ground electrode 16 is flexible. To be flexible, ground electrode 16 is formed of, for example, a thin metal plate. Alternatively, ground electrode 16 may be made of copper foil that is used for a flexible substrate.

Ground electrode 16 is preferably covered by insulator 22a also at surface 16b opposite from surface 16a. Because of this structure, ground electrode 16 is contained inside belt 22. Accordingly, unexpected contact between a metal object and belt 22 can be suppressed. Even in such an event, stable communication is achieved between data communication device 11 and communication control device 1002.

Ground electrode 16 preferably extends to a position close to a leading edge of belt 22. Because of this structure, ground electrode 16 circles the arm when belt 22 is worn around arm 1004a. This structure enables a smaller change of distance between ground electrode 16 and ground electrode 1002b of communication control device 1002 and a smaller change of direction that are not affected by an arm posture including a direction and a position of arm 1004a. Therefore, stable communication is achieved between data communication device 11 and communication control device 1002 without being affected by the arm posture including the direction and position of arm 1004a.

Body case 21 includes fitting case 211 and outer case 212. Communication electrode 15 is disposed in a center of fitting case 211 to be exposed at bottom face 21a. Outer case 212 is disposed on an outer side of fitting case 211. Fitting case 211 and outer case 212 are sealed together. An electric circuit including communication unit 13 and power source 12 is put in an internal space of body case 21.

Belt 22 is inserted between fitting case 211 and outer case 212, thus being joined to body case 21. Fitting case 211 preferably includes, outwardly of an edge of communication electrode 15, frame 21c that is made of a resin. This structure can keep communication electrode 15 and ground electrode 16 apart from each other. Thus communication signal attenuation that might be caused by communication signal flow toward the ground electrode can be suppressed.

Surface 16a of ground electrode 16 is preferably positioned at a distance closer to front face 21d from plane 15b. It is to be noted that front face 21d is a face of body case 21 that is opposite from bottom face 21a. Therefore, a shoulder, for example, is formed between a side edge of bottom face 21a and a belt base where belt 22 is joined to side face 21b of body case 21. Because of this structure, when belt 22 is worn around arm 1004a, belt 22 comes into contact with the arm at a slight distance from an outer edge of bottom face 21a of body case 21. Therefore, when belt 22 is worn around arm 1004a, ground electrode 16 can be restrained from being at a smaller distance from communication electrode 15 near the belt base.

It is preferable that communication electrode 15 be substantially flush with bottom face 21a. Because of this structure, a shorter clearance can be achieved between communication electrode 15 and arm 1004a when data communication device 11 is put on the arm. Communication electrode 15 thus easily comes into contact with arm 1004a. Consequently, during transmission of a communication signal to arm 1004a, communication signal loss can be reduced. It is to be noted that communication electrode 15 may project from bottom face 21a, so that the structure in which communication electrode 15 is substantially flush with bottom face 21a is not limiting. It is also to be noted that communication electrode 15 may be disposed, for example, on the belt side (serving as fitting surface 11a) that faces plane 15b, so that exposing communication electrode 15 at fitting surface 11a of body case 21 is not limiting.

A description is provided next of a structure that enables data communication device 11 to be put on arm 1004a.

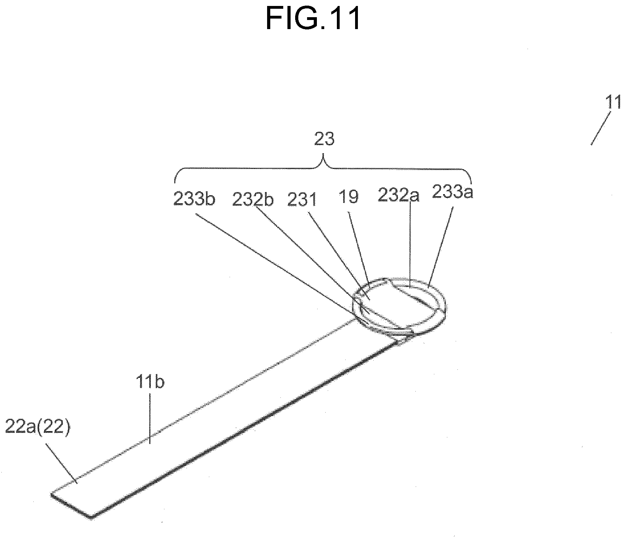

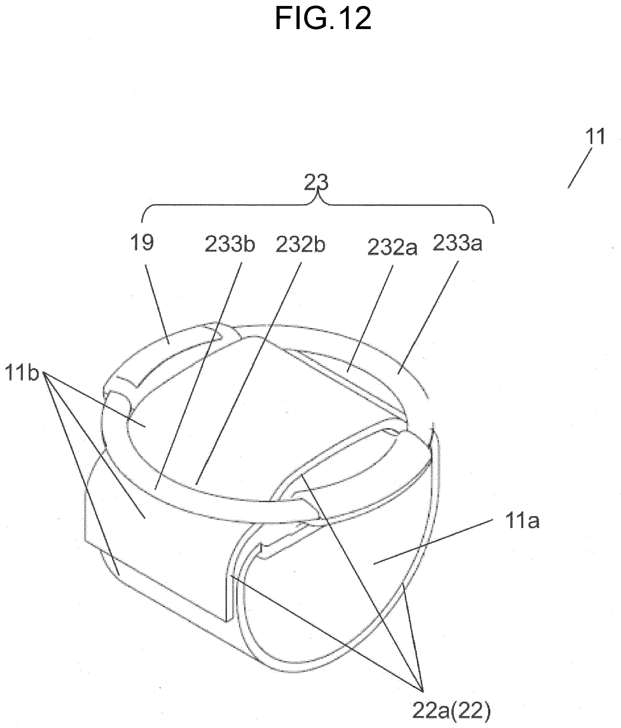

FIG. 11 is a perspective view of data communication device 11. FIG. 12 is a perspective view illustrating data communication device 11 with belt 22 fastened.

The data communication device also includes buckle 23. Buckle 23 is put on with fitting surface 11a circling the arm while facing arm 1004a. Belt 22 has its length adjusted by buckle 23, so that belt 22 can be held around the arm. Data communication device 11 is preferably put on with communication electrode 15 directly touching the human body at its surface 15a. However, every person has a different arm thickness. Therefore, it is desirable that the length of belt 22 be adjusted freely. For that purpose, buckle 23 desirably has a buckle mechanism. As is described below, the buckle mechanism is an internally provided mechanism that immobilizes the belt which is pressed by a pressing device (FIG. 10). This means that buckle 23 does not have a pin that immobilizes belt 22. This also means that belt 22 is not formed with pin holes that each allow passage of the pin to immobilize belt 22. Because the length of belt 22 can be adjusted freely by this mechanism, a reduced clearance can be achieved between communication electrode 15 and arm 1004a no matter how thick arm 1004a can be. Therefore, communication signal loss can be reduced.

If belt 22 is to be formed with those pin holes, ground electrode 16 needs to be formed to avoid the pin holes. In other words, ground electrode 16 has voids that correspond to the pin holes, respectively. However, belt 22 with the buckle mechanism does not have to have the pin holes, so that ground electrode 16 does not have to have those voids corresponding to the pin holes. Therefore, any dimension of ground electrode 16 can be uniform without being affected by the direction of arm 1004a. When seen externally, ground electrode 16 has, in a direction orthogonal to a direction from its base to its leading edge, a width that is uniform from its base to its leading edge.

As illustrated in FIG. 10, buckle 23 having the buckle mechanism includes, for example, buckle body 231, space 232, and an immobilizing assembly 233. Immobilizing assembly 233 is joined to buckle body 231. Space 232 is formed between buckle body 231 and immobilizing assembly 233. When data communication device 11 is put on arm 1004a, the leading edge of belt 22 is inserted through space 232. Immobilizing assembly 233 is pressed against belt 22 in this state. Belt 22 is inserted between immobilizing assembly 233 and buckle body 231, thus being held by this structure.

Buckle 23 is disposed at an outer-surface-end side of body case 21. It is to be noted that body case 21 may also serve as a part of buckle 23. In other words, buckle 23 may include outer case 212 forming buckle body 231, and fitting case 211 having bottom face 21a as fitting surface 11a. In this case, bottom face 21a of fitting case 211 defines fitting surface 11a of buckle 23. Moreover, front face 21d of outer case 212 also serves as a front face of buckle body 231. Having this structure, buckle 23 can have a reduced thickness. Moreover, buckle 23 and body case 21 can be put into one place on belt 22. Therefore, a satisfactory fit is provided when data communication device 11 is put on arm 1004a.

It is to be noted that front face 21d of body case 21 may be independent of buckle body 231, so that the structure in which body case 21 also serves as buckle body 231 is not limiting. In other words, buckle 23 may include buckle body 231, outer case 212, and fitting case 211. In this case, buckle body 231 is fixed to body case 21 while overlapping the outer-surface-end side of body case 21, namely, front face 21d. Because of this structure, buckle 23 can be replaced with ease. Therefore, wearer 1004 can replace buckle 23 with buckle 23 having a design that is to his or her taste. Even in this case, buckle 23 and body case 21 can be put into one place on belt 22.

Body case 21 may be disposed at a different position than buckle 23, so that disposing body case 21 at a fitting-surface-end side of buckle 23 is not limiting. In this case, body case 21 is not disposed to overlap buckle 23. In other words, body case 21 is joined to belt 22 between buckle 23 and the leading edge of belt 22. Belt 22 is joined to buckle 23. Belt 22 is joined to buckle body 231 in this case. In this case, communication electrode 15 may be disposed at a fitting-surface-end side of belt 22 or buckle body 231, so that disposing communication electrode 15 at body case 21 is not limiting. These structures enables buckle 23 to have a reduced thickness. With data communication device 11 being put on arm 1004a, body case 21 attached to arm 1004a preferably faces buckle 23 across arm 1004a. This structure can reduce discomfort when put on arm 1004a.

A detailed description is provided next of immobilizing assembly 233. Immobilizing assembly 233 preferably includes immobilizing part 233a and immobilizing part 233b. Immobilizing parts 233a and 233b are provided side by side in a direction that belt 22 passes. Belt 22 is inserted into space 232a between immobilizing part 233a and buckle body 231 in a direction from fitting surface 11a toward outer surface 11b. Buckle 23 is then inserted into space 232b between immobilizing part 233b and buckle body 231 in a direction from outer surface 11b toward fitting surface 11a. Because of this structure, insertion of belt 22 takes place at two locations: one between immobilizing part 233a and buckle body 231 and the other between immobilizing part 233b and buckle body 231. Therefore, belt 22 is not easily slackened.

The leading edge of ground electrode 16 is provided at an end of belt 22 that is opposite from where belt 22 is joined to body case 21. In other words, ground electrode 16 desirably extends to have its leading edge at the leading edge of belt 22. Having this structure, ground electrode 16 circles arm 1004a. Consequently, stable communication can be achieved without being affected by the direction of arm 1004a.

It is to be noted that ground electrode 16 may extend to where its leading edge at least overlaps buckle 23 with belt 22 being inserted through space 232, so that ground electrode 16 that extends to have its leading edge at the leading edge of belt 22 is not limiting. In this case, outer case 212 or buckle body 231 is preferably made of a metal to be electrically connected to ground electrode 16. Thus outer case 212 or buckle body 231 also functions as a ground electrode. Resulting ground electrode 16 thus formed in this structure can circle arm 1004a. In this case, belt 22 passes by respective outer surfaces of outer case 212 and buckle body 231. Insulator 22a is a covering serving as a surface layer of belt 22. This can mean that there is a smaller possibility of contact between wearer 1004 and outer case 212 or buckle body 231. This can also mean that there is a smaller possibility of contact between a metal object and outer case 212 or buckle body 231. Consequently, stable communication can be achieved.

More desirably, the ground electrode extends so that its leading edge overlaps its base. Ground electrode 16 thus formed to have this structure can circle arm 1004a. In this particular case, because ground electrode 16 formed can circle arm 1004a, each of outer case 212 and buckle body 231 can be made of a resin. If outer case 212 and buckle body 231 are each made of the resin, stable communication can be achieved even when a metal object or the like comes into contact with, for example, outer case 212 or buckle body 231.

### (Variation)

FIG. 25 is a perspective view of data communication device 51 which is a variation. FIG. 26 is an exploded perspective view of data communication device 51. Data communication device 51 is the variation of data communication device 11. Unlike data communication device 11, data communication device 51 includes belt 52 instead of belt 22. Belt 52 differs from belt 22 in that belt 52 connects with two opposite side faces of body case 21. Ground electrode 16 is contained inside belt 52 that extends from both of the side faces of body case 21. In other words, ground electrode 16 extends out of body case 21 in opposite directions. Therefore, body case 21 is disposed at a middle part of belt 52.

Unlike data communication device 11, data communication device 51 includes buckle 53 instead of buckle 23. Buckle 53 is a pin type buckle. In this case, buckle 53 includes ring 531, opening 532, and pin holes 533. Ring 531 is formed at one end of belt 52. Ring 531 has opening 532 that allows passage of the belt. Pin holes 533 are formed between ring 531 and body case 21. An opposite end of belt 52, on the other hand, has pin 534. It is to be noted here that belt 52 has the plurality of pin holes 533. Therefore, pin 534 is inserted into any one of the plurality of pin holes 533 to adjust length of belt 52. In other words, wearer 1004 inserts pin 534 into pin hole 533 that is right for his or her arm thickness to fit data communication device 51 to arm 1004a.

Ground electrode 54 is contained inside belt 52. Ground electrode 54 here has slit 541 corresponding to an area formed with the plurality of pin holes 533.

The above exemplary embodiment has been described above as being illustrative of the technique of the present disclosure. However, the technique of the present disclosure is not limited to the above exemplary embodiment and is also applicable to exemplary embodiments including appropriate changes, replacements, additions, and omissions. Moreover, the constituent elements described in the exemplary embodiment can be combined in a new exemplary embodiment.

### INDUSTRIAL APPLICABILITY

A data communication device according to the present disclosure is effective in achieving stable communication and thus is useful as a device that is fitted to a human body for intra-body communication.

### REFERENCE MARKS IN THE DRAWINGS

- 11, 51:: data communication device
- 11a:: fitting surface
- 11b:: outer surface
- 12:: power source
- 13:: communication unit
- 13a:: transmitter
- 13b:: receiver
- 13c:: control circuit
- 13d:: memory
- 14:: communication antenna
- 15:: communication electrode
- 15a:: surface
- 15b:: plane
- 16, 54:: ground electrode
- 16a:: surface
- 16b:: surface
- 17:: ground
- 18:: external input and output interface
- 19:: display unit
- 21:: body case
- 21a:: bottom face
- 21b:: side face
- 21c:: frame
- 21d:: front face
- 22, 52:: belt
- 22a:: insulator
- 23, 53:: buckle
- 211:: fitting case
- 212:: outer case
- 231:: buckle body
- 232:: space
- 232a:: space
- 232b:: space
- 233:: immobilizing assembly
- 233a, 233b:: immobilizing part
- 531:: ring
- 532:: opening
- 533:: pin hole
- 534:: pin
- 541:: slit
- 1001:: data communication system
- 1002:: communication control device
- 1002a:: communication electrode
- 1002b:: ground electrode
- 1002c:: communication unit
- 1003:: ground
- 1004:: wearer
- 1004a:: arm
- 1004b:: finger
- 1004c:: hand

## Claims

1. A data communication device comprising:
a belt;
a buckle joined to the belt, the buckle including a fitting surface;
a communication electrode disposed at the buckle, the communication electrode being exposed at the fitting surface; and
a communication unit disposed at the buckle, the communication unit being electrically connected to the communication electrode,
wherein the buckle includes a buckle mechanism.

2. The data communication device according to claim 1, further comprising a body case that includes an outer surface opposing the fitting surface,
wherein the buckle is provided at the outer surface of the body case, and
wherein the communication electrode is exposed at the body case.

3. The data communication device according to claim 2, wherein the body case also serves as a part of the buckle.

4. The data communication device according to claim 1, wherein:
the buckle includes:
a buckle body;
an immobilizing assembly joined to the buckle body; and
a space that allows a leading edge of the belt to pass through, the space being formed between the immobilizing assembly and the buckle body; and
the immobilizing assembly presses the belt when the belt is inserted through the space.

5. The data communication device according to claim 4, wherein the immobilizing assembly includes a first immobilizing part and a second immobilizing part that are provided side by side in a direction that the belt is inserted.

6. The data communication device according to claim 4, further comprising a ground electrode including one end and an opposite end, the one end being electrically connected to a ground of the communication unit inside the body case, the opposite end having a leading edge, the ground electrode being contained inside the belt,
wherein the ground electrode extends to where the leading edge of the ground electrode overlaps the buckle with the belt being inserted through the sp ace.

7. The data communication device according to claim 6, wherein the ground electrode extending has a leading edge at a position where the buckle is joined to the belt.

8. The data communication device according to claim 6, wherein the ground electrode has a uniform width.

9. A data communication device comprising:
a body case including a bottom face and a side face;
a belt joined to the body case at the side face of the body case;
a power source housed in the body case;
a communication unit that is driven by the power source;
a communication electrode connected to the communication unit, the communication electrode being exposed at the bottom face of the body case; and
a ground electrode that is plate-shaped, the ground electrode being connected to a ground of the communication unit inside the body case,
wherein the belt at least partly includes an insulator,
wherein the ground electrode includes a second surface covered by the insulator, and
wherein the second surface extends out of the body case to face a first plane extending, as an extension of a surface of the communication electrode, beyond the body case.

10. The data communication device according to claim 9, wherein the ground electrode includes a third surface opposite from the second surface and is contained inside the belt with the third surface being covered by the insulator.

11. The data communication device according to claim 10, wherein the ground electrode extends to a position close to a leading edge of the belt.

12. The data communication device according to claim 9, wherein the body case includes, outwardly of an edge of the communication electrode, a frame comprising a resin.

13. The data communication device according to claim 9, wherein:
the body case includes a front face opposite from a bottom face; and
the second surface of the ground electrode is positioned at a distance closer to the front face from the first plane.

14. The data communication device according to claim 9, wherein the communication electrode is substantially flush with the bottom face or projects from the bottom face.
